# EUROPEAN PATENT APPLICATION

(11) **EP 0 895 986 A2**
(43) Date of publication of application: **10.02.1999**
(21) Application number: 98306299.3
(22) Date of filing: 06.08.1998
(51) Int. Cl.: C07C 253/20, C07C 255/50

(54) **A process for producing a nitrile**

(30) Priority: 06.08.1997 JP 212249/97
(71) Applicant: TORAY INDUSTRIES, INC., Urayasu Chiba 279-8555 (JP)
(72) Inventor: Matsuoka, Shotaro, Nagoya-shi, Aichi 467-0043 (JP); Sematsu, Masaaki, Nisshin-shi, Aichi 470-0134 (JP); Ishikawa, Mamoru, Kasatsu-shi, Shiga 525-0045 (JP)
(74) Representative: Coleiro, Raymond

(57) **Abstract**

In a process for producing a nitrile (II) :

R - CN (II)

(where R is optionally substituted alkyl, alkenyl, alkynyl, aryl or aralkyl) by dehydration of an amide (I):

R - CONH₂ (I)

(where R is as defined above), the dehydration reaction is carried out in the presence of a carboxylic acid (III) :

R - COOH (III)

(where R is as defined above).

## Description

The present invention relates to a process for producing a nitrile at high yield industrially safely from an amide or from the reaction between urea and a carboxylic acid. In more detail, the present invention relates to a process for producing a nitrile represented by the following formula (II):

R - CN (II)

(where R is a substituted or unsubstituted alkyl group, alkenyl group, alkynyl group, aryl group, or aralkyl group), by dehydration of an amide represented by the following formula (I): 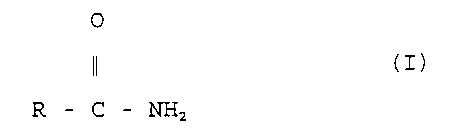 (where R is as defined above) in the presence of a carboxylic acid represented by the following formula (III):

R - COOH (III)

(where R is as defined above).

Such nitriles are compounds very important as intermediate products from which, for example, medicines, chemicals for use in agriculture, dyes and pigments, can be prepared.

Various nitrile production processes are known, and processes from the production of nitriles by the dehydration of an amide are generally known. These processes are proposed, for example, in JP-B-50-030607, JP-B-53-023819, JP-B-53-023820, JP-B-53-023821, JP-A-50-013326, JP-A-62-167749, JP-A-62-289552 and JP-A-02-295957.

Processes for producing nitriles by the reaction between a carboxylic acid and urea are also proposed, for example, in JP-B-62-005899, JP-A-54-095541 and Organic Synthesis Collective (1963),4, 513.

However, these processes were found to present several problems for industrialization. For example, the dehydration reaction of an amide has a disadvantage in that since the water produced simultaneously causes a hydrolysis reaction with the raw amide or the produced nitrile, to produce a carboxylic acid, the yield of the intended nitrile cannot be raised.

Furthermore, in a process to obtain a nitrile from the reaction between a carboxylic acid and urea, the carboxylic acid and urea are supplied beforehand, that is, the carboxylic acid and urea are arranged to be present before the start of reaction, to avoid the trouble of adding urea or to improve the carboxylic acid dissolving temperature. However, in such an operation, which allows both the compounds to coexist, a sudden reaction occurs between the carboxylic acid and urea, causing the reaction mixture to bump, or an exhaust gas of carbon dioxide, ammonia, etc. is generated suddenly in a large amount, with resultant inconvenience to the operators and an adverse effect upon safety and the environment, such problems being difficult to overcome. In theory, it is possible to use an excessive amount of urea against the carboxylic acid, for almost completely consuming the raw carboxylic acid and for increasing the production of an amide as an intermediate product and furthermore for increasing the production of a nitrile from the subsequent dehydration reaction. However, in this case, the urea not used for the reaction is self-decomposed to give a sublimate which plugs the exhaust gas line connected to the reactor, which is disadvantageous having regard to both working convenience and safety.

Moreover, when a nitrile is obtained from the reaction between a carboxylic acid and urea, water, carbon dioxide and ammonia are produced simultaneously, and they are discharged outside the system from the reactor through an exhaust gas distillate line. In this case, a sublimate such as ammonium bicarbonate, or a decomposition product of urea itself, are discharged simultaneously, to plug the exhaust gas line, thus raising the internal pressure of the reactor, which is a problem threatening safety, and frequent unplugging work is necessitated, these being disadvantages which seriously inconvenience operators during production.

Furthermore, in the amide dehydration reaction, if byproduced water is distilled out of the reactor, the equilibrium shifts toward the product side, to ensure smooth progression of reaction, and for more advantageous production of a nitrile, it is necessary to distill out the produced nitrile together with byproduced water from the reactor, for further shifting the equilibrium toward the product side, to ensure more smooth progression of reaction. In this case, the nitrile can also be isolated without thermal deterioration. However, on the other hand, if the nitrile and byproduced water are not smoothly distilled out of the reactor, the reaction rate becomes low, and in addition, the nitrile retained in the reactor reacts with byproduced water, to be converted into the corresponding carboxylic acid, thus considerably decreasing the nitrile production.

Therefore, these conventional nitrile production processes are not sufficiently satisfactory for application industrially.

The present invention addresses the problem of providing a process for producing a nitrile at a high yield without any problem in securing safety from the reaction for dehydration of an amide or the reaction between urea and a carboxylic acid.

After intensive studies directed to solving the above problems, we found surprisingly that if the dehydration reaction of an amide is effected in the presence of a carboxylic acid corresponding to the amide, the byproduction of the carboxylic acid is inhibited, while the yield of the intended nitrile improves.

It was also found that if urea is added to the carboxylic acid when the carboxylic acid is molten or completely dissolved in a solvent, the corresponding nitrile can be produced stably without bumping of the reaction mixture caused by the sudden reaction between the carboxylic acid and urea or without the sudden mass generation of exhaust gas of carbon dioxide, ammonia, etc.

It was also found that if the amount of urea used is controlled at less than 0.8 : 1 in molar ratio to the carboxylic acid, the nitrile can be stably produced without any inconvenient plugging in the exhaust gas line.

It was also found that if the exhaust gas distillate line connected to the reactor is kept at 60°C or higher, almost nothing plugs the line, which solves problems which would otherwise render difficult meeting the requirement to secure safety and which would otherwise render the working operation especially inconvenient to perform.

It was also found that if the produced nitrile is distilled out while a gas inactive to the amide dehydration reaction is fed in during the reaction, the produced nitrile and byproduced water can be smoothly distilled out, and that the intended nitrile can be obtained at a high yield.

A process according to one aspect of the invention for producing a nitrile, in which the dehydration reaction of an amide represented by the following formula (I) 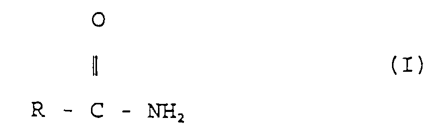 (where R is a substituted or unsubstituted alkyl group, alkenyl group, alkynyl group, aryl group or aralkyl group) is effected to produce a nitrile represented by the following formula (II)

R - CN (II)

(where R is as defined above), is characterized in that the dehydration reaction is effected in the presence of a carboxylic acid represented by the following formula (III) :

R - COOH (III)

(where R is as defined above).

As a preferred embodiment of this aspect of the present invention, the dehydration of an amide represented by the formula (I) is effected in the presence of the residue remaining after preparing, and more preferably after removing, the nitrile represented by the formula (II), which removal is preferably effected by distillation.

Furthermore, a process according to another aspect of the invention for producing a nitrile, in which an amide represented by the following formula (I): 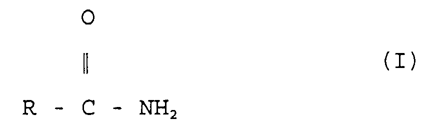 (where R is as defined above) is obtained by reaction between a carboxylic acid represented by the following formula (III):

R - COOH (III)

(where R is as defined above) and urea, and subsequently dehydration of the resultant amide represented by the formula (I) is effected to produce a nitrile represented by the following formula (II):

R - CN (II)

(where R is as defined above), is characterized in that the conversion percentage of the carboxylic acid represented by the formula (III) is less than 100%, and that the dehydration reaction is effected while the carboxylic acid represented by the formula (III) remains in the produced amide.

In this aspect of the present invention, it is preferable that urea is added to the carboxylic acid represented by the formula (III) when the carboxylic acid is molten or that urea is added to the carboxylic acid represented by the formula (III) when the caboxylic acid is completely dissolved in a solvent, and that the amount of urea used is less than 0.8 : 1 in molar ratio to the carboxylic acid.

Furthermore, in the present invention, it is preferable that while a gas inactive to the amide dehydration reaction is fed into the reaction vessel during the reaction, the nitrile and water produced are distilled out, that the gas inactive to the reaction is any one of nitrogen, helium, argon or carbon dioxide, and that each R in the compounds represented by the formulae (I), (II) and (III) are aryl groups.

Furthermore, in a process embodying the present invention, it is preferable that an inorganic acid and/or a cobalt salt is used as a catalyst, and that the exhaust gas distillate line connected to the reactor for discharging the byproducts of the reaction is kept at 60°C or higher.

Embodiments of the present invention are described below in detail.

In the process of the invention for producing a nitrile by dehydration of an amide, an amide represented by the following formula (I): 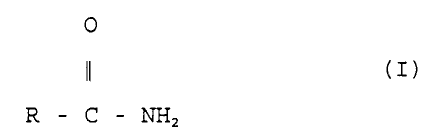 (where R is a substituted or unsubstituted alkyl group, alkenyl group, alkynyl group, aryl group or aralkyl group) is used as a raw material. In the formula, it is preferable that R is a substituted or unsubstituted C₁₋₂₀ alkyl group, C₂₋₂₀ alkenyl group or C₂₋₂₀ alkynyl group, or a substituted or unsubstituted C₆₋₂₀ aryl group or a substituted or unsubstituted C₆₋₂₀ aralkyl group.

Above all, an amide in which R of the formula (I) is a substituted or unsubstituted C₆₋₂₀ aryl group is more preferable, and furthermore, an amide represented by the following formula (IV): 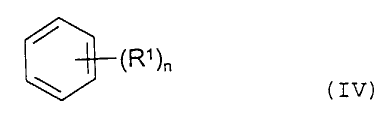 (where R¹ stands for a C₁₋₄ alkyl group, C₁₋₄ alkoxy group or a halogen atom, and n is 0,1 or 2) is preferable.

The amides which can be used here include, for example, aliphatic amides such as acetic amide, valeric amide, caproic amide, lauric amide, stearic amide and oleic amide, aromatic amides such as benzamide, toluamide, ethylbenzamide, chlorobenzamide, dichlorobenzamide, bromobenzamide, dibromobenzamide, methoxybenzamide, ethoxybenzamide, dimethoxybenzamide, nitrobenzamide, cyanobenzamide, aminobenzamide, hydroxybenzamide, dihydroxybenzamide, hydroxymethylbenzamide, formylbenzamide, phthalamide and naphthoamide.

These amides can be easily produced, for example, by reaction between a carboxylic acid and ammonia or between a carboxylic acid and urea or ammonium carbonate, to produce ammonia under the reaction conditions, or by reaction between a carboxylic acid halide and ammonia. In processes embodying the present invention, any of the amides obtained by the above methods can be used after it has been purified or as it is without being purified, without any problem.

The amide dehydration reaction in the process of the present invention is effected in the presence of a carboxylic acid represented by the following formula (III):

R-COOH (III)

(where R is as defined above). It is preferable that R is a substituted or unsubstituted C₁₋₂₀ alkyl group, a C₂₋₂₀ alkenyl group, a C₂₋₂₀ alkynyl group, or a substituted or unsubstituted C₆₋₂₀ aryl group or a substituted or unsubstituted C₆₋₂₀ aralkyl group.

specifically, the dehydration reaction of an amide is effected in the presence of an aliphatic carboxylic acid such as acetic acid, valeric acid, caproic acid, lauric acid, stearic acid, or oleic acid, or an aromatic carboxylic acid such as benzoic acid, toluic acid, ethylbenzoic acid, chlorobenzoic acid, dichlorobenzoic acid, bromobenzoic acid, dibromobenzoic acid, anisic acid, ethoxybenzoic acid, dimethoxybenzoic acid, nitrobenzoic acid, cayanobenzoic acid, aminobenzoic acid, hydroxybenzoic acid, dihydroxybenzoic acid, hydroxymethylbenzoic acid, formyl benzoic acid, phthalic acid or naphthylic acid. These carboxylic acids can be easily produced by publicly known techniques such as vapor phase or liquid phase air oxidation, nitric acid oxidation, electrolytic oxidation and reagent oxidation.

In processes embodying the present invention, to provide the carboxylic acid, the carboxylic acid can be supplied together with the raw material, or can be added during reaction. Since the residue remaining after removing the nitrile obtained by the amidation reaction mainly contains a byproduced carboxylic acid, it can be recycled. That is, the amide dehydration reaction can also be effected in the presence of the residue.

In the amide dehydration reaction in a process embodying the present invention, as the amide used as the raw material, an amide produced from a carboxylic acid according to a publicly known technique can also be used, as described above. In this case, the conversion percentage of the carboxylic acid is kept low, to leave the carboxylic acid in the produced amide, in order to use the reaction mixture as it is for the dehydration reaction. There are several publicly known techniques for obtaining an amide from a carboxylic acid, as described above. An especially preferred method is to obtain an amide by the reaction between a carboxylic acid and urea. In this case, the conversion percentage of the carboxylic acid should be less than 100 mol%. A preferred range is 50 to 99 mol%, and a more preferred range is 60 to 95 mol%.

The amount of the carboxylic acid used in the dehydration reaction of the present invention is preferably 1 to 50 wt% base on the weight of the raw amide. A more preferred range is 5 to 30 wt%.

The dehydration reaction of the present invention can also be effected in the presence of a catalyst such as an inorganic acid or cobalt salt. The inorganic acids which can be used here include boric acid, phosphoric acid, phosphorous acid and sulfuric acid. The cobalt salts which can be used here include cobalt borate, cobalt acetate, cobalt oxide, cobalt chloride and cobalt sulfate. One or more as a mixture of these compounds can be used. It is preferable to use cobalt borate or a mixtre of boric acid and cobalt acetate.

It is preferable that the amount of the catalyst is 0.01 to 30 wt% based on the weight of the amide. A more preferred range is about 0.1 to 10 wt%.

The temperature of the dehydration reaction of the present invention depends on the compounds used, but is usually 200 to 400°C. A preferred range is 220 to 300°C. The reaction can be effected at atmospheric pressure, with pressurization or under reduced pressure. It is preferable that the reaction is effected at atmospheric pressure or under reduced pressure. The reaction can also be effected in vapor phase or liquid phase. The amide and the carboxylic acid can be rendered molten as they are for reaction, or can be dissolved into a solvent for reaction. Whether the reaction is a batch or continuous reaction, no particular inconvenience is caused.

In the present invention, in the process for producing a nitrile by the reaction between a carboxylic acid and urea, a carboxylic acid represented by the following formula (III):

R-COOH (III)

(where R is a substituted or unsubstituted alkyl group, alkenyl group, alkynyl group, aryl group or aralkyl group) is used as a raw material. It is preferable that R is a substituted or unsubstituted C₁₋₂₀ alkyl group, a C₂₋₂₀ alkenyl group, or C₂₋₂₀ alkynyl group, or a substituted or unsubstituted C₆₋₂₀ aralkyl group). Specifically the carboxylic acids which can be used here include aliphatic carboxylic acids such as lauric acid, stearic acid and oleic acid, aromatic carboxylic acids such as benzoic acid, toluic acid, ethylbenzoic acid, chlorobenzoic acid, dichlorobenzoic acid, bromobenzoic acid, dibromobenzoic acid, anisic acid, ethoxybenzoic acid, diemthoxybenzoic acid, nitrobenzoic acid, cyanobenzoic acid, aminobenzoic acid, hydroxybenzoic acid, dihydroxybenzoic acid, hydroxymethylbenzoic acid, formylbenzoic acid, phthalic acid and naphthylic acid.

These carboxylic acids can be easily produced by publicly known techniques such as vapor phase or liquid phase air oxidation, nitric acid oxidation, electrolytic oxidation and reagent oxidation. As the carboxylic acid used as the raw material, a product obtained by any of the above techniques can be used after it has been purified or as it is without being purified, without any problem.

In a process embodying the present invention, from the reaction between a carboxylic acid and urea, an amide represented by the following formula (I): 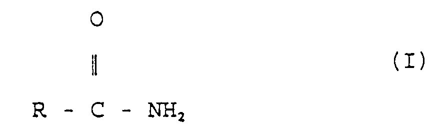 (where R is a substituted or unsubstituted alkyl group, alkenyl group, alkynyl group, aryl group or aralkyl group) is at first produced, and in succession, by the amide dehydration reaction, the intended nitrile can be produced. In this case, it is preferable that R is a substituted or unsubstituted C₁₋₂₀ alkyl group, a C₂₋₂₀ alkenyl group, C₂₋₂₀ alkynyl group, or a substituted or unsubstituted C₆₋₂₀ aryl group or a substituted or unsubstituted C₆₋₂₀ aralkyl group.

The amide produced as an intermediate product can be isolated and purified to be used for the dehydration reaction, or the crude product can be used as it is. Usually, the crude product is directly used for the dehydration reaction.

In the reaction between a carboxylic acid and urea in a process embodying the present invention, it is preferable that urea is added to the carboxylic acid at a rate such as not to cause sudden reaction with the carboxylic acid, when the carboxylic aid is molten or completely dissolved in a solvent. Urea can be added as a solid or in a molten state or as a solution in a solvent inactive to the reaction.

As the solvent used in the reaction between carboxylic acid and urea in a process embodying the present invention, any solvent can be used without any inconvenience as far as it is inactive to the reaction between urea and a carboxylic acid. The solvents which can be used here include aromatic or aliphatic hydrocarbons such as cumene, trimethylbenzene, tetratmethylbenzene, cymene, diisopropylbenzene, decalin, tetralin, methylnaphthalene, dimethylnaphthalene, isopropylnaphthalene and diisopropylnaphthalene, aromatic chlorides such as chlorotoluene, dichlorotoluene, dichlorobenzene and trichlorobenzene, aromatic nitro compounds such as nitrobenzene and nitrotoluene, aromatic ether compounds such as diphenyl ether, amide compounds such as dimethylformamide and dimethylacetamide and sulfur-containing compounds such as dimethyl sulfoxide. The amount of the solvent used is not especially limited either, as far as the raw carboxylic acid can be dissolved in the reaction conditions. As the solvent, a pure product can be used or a solvent recovered from the reaction can also be used without any problem.

It is preferable that the amount of urea used in the reaction between a carboxylic acid and urea of the present invention is less than 0.8 : 1 in molar ratio to the carboxylic acid. A more preferable range is 0.5 : 1 to 0.8 : 1.

In the reaction between a carboxylic acid and urea of the present invention, the exhaust gas distillate line connected to the reactor is preferably a pipe connecting the reactor with a scrubber for catching the exhaust gas, and through the line, the carbon dioxide, water and ammonia byproduced during the reaction, sublimate such as ammonium bicarbonate, decomposition product of urea itself, etc. are discharged. As the material of the pipe, any material can be used without any inconvenience as far as it can resist the compounds discharged.

The heating temperature of the exhaust gas distillate line connected to the reactor should not be lower than 60°C which is the sublimation temperature of ammonium bicarbonate mainly contained in the plugging material. A preferred range is 60 to 250°C, and a more preferred range is 100 to 220°C. Any heating method can be used without any problem, as far as the exhaust gas line can be kept at 60°C or higher.

The reaction between a carboxylic acid and urea in a process embodying the present invention can be effected in the presence of a catalyst such as an inorganic acid or cobalt salt. The inorganic acids which can be used here include boric acid, phosphoric acid, phosphorous acid and sulfuric acid. The cobalt salts which can be used here include cobalt borate, cobalt acetate, cobalt oxide, cobalt chloride and cobalt sulfate.

One or more as a mixture of these compounds can be used. It is preferable to use cobalt borate or a mixture of boric acid and cobalt acetate. It is preferable that the amount of the catalyst is 0.01 to 30 wt% based on the weight of the amide. A more preferred range is about 0.1 to 10 wt%.

The reaction temperature of the reaction between a carboxylic acid and urea in a process embodying the present invention depends on the compounds used. It is preferable that the first amidation is effected at 105 to 350°C. A more preferred range us 170 to 250°C. It is preferable that the temperature of the subsequent nitrilation is 200 to 400°C. A more preferred range is 220 to 300°C.

The reaction can be effected at atmospheric pressure, with pressurization or under reduced pressure. It is preferable that the first amidation is effected at atmospheric pressure or with pressurization, and that the subsequent nitrilation is effected at atmospheric pressure or under reduced pressure.

The reaction can be effected in a vapor phase or liquid phase, but it is preferable that the reaction is effected in a liquid phase. Whether the reaction is a batch or continuous reaction, no particular inconvenience is caused.

In a process embodying the present invention, as described above, the product of the reaction between a carboxylic acid and urea is also the starting material for the amide dehydration reaction in the stage of nitrilation. Because of the dehydration reaction, it is preferable to effect the reaction while removing the produced water. For more advantageous production of a nitrile, as described above, the produced nitrile should be distilled out together with byproduced water from the reactor during reaction. For this purpose, it is preferable to feed into the reaction vessel a gas inactive to the amide dehydration reaction. Any gas inactive to the reaction can be used without any special restriction, and for example, nitrogen, helium, argon or carbon dioxide, can be used. The gas flow rate is preferably about 1 to 300 ml/min per mole of the amide.

The nitrile obtained in this manner by the production process of the present invention can be purified by a conventional method such as distillation or recrystallization.

According to a nitrile production process embodying the present invention, a nitrile can be produced at a high yield without any problem in security of safety from the amide dehydration reaction or from the reaction between urea or a carboxylic acid.

### Examples

Preferred embodiments of the present invention will now be described in more detail with reference to the following Examples.

### Example 1

A 200 ml four-neck flask equipped with a stirrer, thermometer, gas blow pipe and distillate pipe was charged with 80 g of p-toluamide (0.59 mol; produced by Tokyo Kasei), 20 g of p-toluic acid (015 mol; guaranteed reagent, produced by Tokyo Kasei) and 1.60 of cobalt acetate tetrahydrate (2.0 wt% based on the weight of p-toluamide; 1st grade reagent, produced by Katayma Kagaku), and rection was effected at a reaction temperature of 240°C for 9 hours while nitrogen was blown in at 80 ml/min. After completion of reaction, the distillate and the residue in the flask were analyzed by high performance liquid chromatography, and p-toluamide conversion percentage was found to be 97.8%, p-tolunitrile production percentage, 74.4%, and p-toluic acid byproduction percentage, 13.9%.

### Example 2

Reaction was effected as described in Example 1, except that 80 g of p-toluamide, 0.32 g of cobalt acetate tetrahydrate and 0.32 g of boric acid were added to 31.3 g of the residue (containing 22.2 g of p-toluic acid, 1.5 g of p-tolunitrile, 1.1 g of p-toluamide and 2.56 g of catalyst) remaining after distilling out p-tolunitrile in Example 1. As a result, p-toluamide conversion percentage was 94.5%, p-tolunitrile production percentage, 82.9%, and p-toluic acid byproduction percentage, 10.6%.

### Example 3

An exhaust gas distillate pipe heated to 120°C by a ribbon heater in the range up to a water-filled trap was installed in a 200 ml four-neck flask equipped with a stirrer, thermometer, gas blow pipe and distillate pipe. Into the flask were supplied 100 g of p-toluic acid (0.73 mol; guaranteed reagent, produced by Tokyo Kasei), 2.00 g of cobalt acetace tetrahydrate (1.9 wt% based on the weight of p-toluamide; guaranteed reagent, produced by Wako Junyaku) and 2.00 g of boric acid (1.9 wt% based on the weight of p-toluamide; 1st grade reagent, produced by Katayama Kagaku). Reaction was effected at a reaction temperature of 190°C while 33.15 g of urea (0.55 mol; 0.75 in molar ratio to p-toluic acid) was added, taking 6.75 hours, and furthermore, the reaction mixture was stirred at 190°C for 1.25 hours. The product was analyzed by high performance liquid chromatography, and p-toluic acid conversion percentage was found to be 85.5%, p-toluamide production percentage, 79.4% and p-tolunitrile production percentage, 1.5%. The product was heated to 240°C, and reaction was effected for 9 hours while nitrogen was blown in at 80 ml/min. After completion of reaction, the distillate and the residue in the flask were analyzed by high performance liquid chromatography, and p-toluic acid conversion percentage was found co be 91.5%, p-tolunitrile production percentage, 85.2%, and p-toluamide byproduction percentage, 1.3%.

### Example 4

Reaction was effected as described in Example 1, except that nitrogen flow rate was 25 ml/min. As a result, the reaction time was 20 hours, and p-toluamide conversion percentage was 94.5%, p-tolunitrile production percentage, 82.9%, and p-toluic acid byproduction percentage, 10.6%.

### Comparative Example 1

Reaction was effected as described in Example 1, except that p-toluamide only was supplied in an amount of 100 g, 5 without adding p-toluic acid. As a result, p-toluamide conversion percentage was 88.7%, p-tolunitrile production percentage, 57.8%, and p-toluic acid byproduction percentage, 27.1%.

### Comparative Example 2

Reaction was effected as described in Example 3, except that 100 g of p-toluic acid and 18 g of urea (0.30 mol) were supplied, and that reaction was effected with the temperature raised gradually from the melting point of urea, i.e., 130°C to 190°C while 40 g of urea (0.67 mol) was added little by little. However, due to sudden reaction halfway, the reaction mixture bumped, and the distillate pipe was plugged while a strong irritating odor of ammonia was generated. Because of these problems, it became difficult in view of safety to effect the reaction, and the reaction was terminated.

### Comparative Example 3

Reaction was effected as described in Example 1, except that nitrogen was not fed. As a result, almost nothing was distilled even after a lapse of 7 hours. Thus, the reaction was given up. The residue was analyzed, and p-toluamide conversion percentage was found to be 38.4%, p-tolunitrile production percentage 16.7%, and p-toluic acid byproduction percentage 22.4%.

## Claims

1. A process for producing a nitrile represented by the following formula (II):
R - CN (II)
(where R is a substituted or unsubstituted alkyl group, alkenyl group, alkynyl group, aryl group or aralkyl group) by dehydration of an amide represented by the following formula (I): 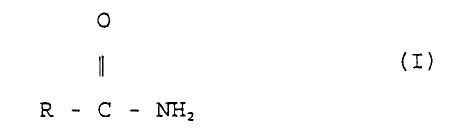 (where R is as defined above), characterised in that the dehydration of the amide is effected in the presence of a carboxylic acid represented by the following formula (III) :
R - COOH (III)
(where R is as defined above).

2. A process according to claim 1, wherein the dehydration of the amide represented by the formula (I) is effected in the presence of the residue remaining after preparing the nitrile represented by the formula (II).

3. A process for producing a nitrile, in which an amide represented by the following formula (I): 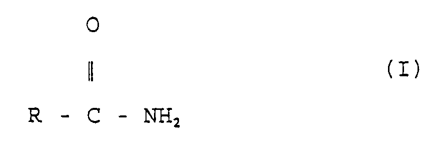 (where R is as defined in claim 1) is obtained by reaction between a carboxylic acid represented by the following formula (III):
R - COOH (III)
(where R is as defined in claim 1) and urea, and subsequently a nitrile represented by the following formula (II):
R - CN (II)
(where R stands for a substituted or unsubstituted alkyl group, alkenyl group, alkynyl group, aryl group or aralkyl group) is produced by dehydration of the resultant amide represented by the formula (I), characterized in that the conversion percentage of the carboxylic acid represented by the formula (III) is less than 100% and that the dehydration reaction is effected while the carboxylic acid represented by formula (III) remains in the produced amide.

4. A process according to claim 3, wherein urea is added to the carboxylic acid represented by the formula (III) when the carboxylic acid is molten.

5. A process according to claim 3, wherein urea is added to the carboxylic acid represented by the formula (III) when the carboxylic acid is completely dissolved in a solvent.

6. A process according to any one of claims 3 to 5, wherein the amount of urea used is less than 0.8 : 1 in molar ratio to the carboxylic acid.

7. A process according to any one of claims 1 to 6, wherein, in the formula (I), R is a substituted or unsubstituted C₆₋₂₀ aryl group.

8. A process according to any one of claims 1 to 6, wherein the amide represented by the formula (I) is an amide represented by the following formula (IV): 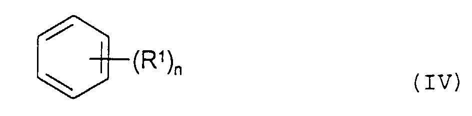 (where R¹ is a C₁₋₄ alkyl group, a C₁₋₄ alkoxy group or a halogen atom, and n is 0, 1 or 2).

9. A process according to any one of claims 1 to 8, wherein a gas inactive to the dehydration of the amide is fed into the reaction vessel during the reaction, while simultaneously with the feeding gas the resultant nitrile and water are distilled out.

10. A process according to claim 9, wherein the gas inactive to the dehydration reaction is any one of nitrogen, helium, argon and carbon dioxide.

11. A process according to any one of claims 1 to 10, wherein each R in the compounds represented by the formulae (I), (II) and (III) is an aryl group.

12. A process according to any one of claims 1 to 11, wherein an inorganic acid and/or cobalt salt is used as a catalyst.

13. A process according to any one of claims 1 to 12, wherein an exhaust gas distillate line connected to the reactor for discharging the byproducts of the reaction is kept at a temperature of 60°C or higher.
